# EUROPEAN PATENT SPECIFICATION

(11) **EP 0 812 580 B1**
(45) Date of publication and mention of the grant of the patent: **18.02.2004**
(21) Application number: 97304096.7
(22) Date of filing: 12.06.1997
(51) Int. Cl.: A61F 2/06

(54) **A stent**
Stent
Prothèse endovasculaire

(30) Priority: 13.06.1996 US 662476; 22.10.1996 US 735022
(43) Date of publication of application: 17.12.1997
(73) Proprietor: Nitinol Devices & Components Inc., Fremont, CA 94539 (US)
(72) Inventor: Stöckel, Dieter, Los Altos, CA-94024 (US); Burpee, Janet, Santa Clara, CA-95050 (US); Duerig, Thomas, Fremont, CA 94539 (US)
(74) Representative: Belcher, Simon James

(56) References cited:
- EP-A- 0 566 807
- EP-A- 0 669 114
- WO-A-96/14029
- WO-A-97/14375
- US-A- 5 290 305

## Description

This invention relates to a stent.

Stents are used in lumens in a human or animal body. When properly positioned in a lumen, a stent can contact the wall of the lumen to support it or to force the wall outwardly.

A stent can be made from a material which enables the stent to be compressed transversely elastically so that it can then recover outwardly when the compressing force is removed, into contact with the wall of the lumen. Such stents are often referred to as "self-expanding stents", and differ from other stents such as balloon expandable stents which are plastically expanded against a lumen wall by inflating an internal balloon. The enhanced elastic properties available from shape memory alloys as a result of a transformation between martensite and austenite phases of the alloys make them particularly well suited to use in self-expanding stents. The nature of the superelastic transformations of shape memory alloys is discussed in "Engineering Aspects of Shape Memory Alloys", T W Duerig et al, on page 370, Butterworth-Heinemann (1990). A principal transformation of shape memory alloys involves an initial increase in strain, approximately linearly with stress. This behaviour is reversible, and corresponds to conventional elastic deformation. Subsequent increases in strain are accompanied by little or no increase in stress, over a limited range of strain to the end of the "loading plateau". The loading plateau stress is defined by the inflection point on the stress/strain graph. Subsequent increases in strain are accompanied by increases in stress. On unloading, there is a decline in stress with reducing strain to the start of the "unloading plateau" evidenced by the existence of an inflection point along which stress changes little with reducing strain. At the end of the unloading plateau, stress reduces with reducing strain. The unloading plateau stress is also defined by the inflection point on the stress/strain graph. Any residual strain after unloading to zero stress is the permanent set of the sample. Characteristics of this deformation, the loading plateau, the unloading plateau, the elastic modulus, the plateau length and the permanent set (defined with respect to a specific total deformation) are established, and are defined in, for example, "Engineering Aspects of Shape Memory Alloys", on page 376.

Non-linear superelastic properties can be introduced in a shape memory alloy by a process which involves cold working the alloy, for example by a process that involves pressing, swaging or drawing. The cold working step is followed by an annealing step while the component is restrained at a temperature that is sufficiently high to cause dislocations introduced by the cold working to combine and dislocations to align.

The technique for introducing superelastic properties can be varied from that described above. For example, instead of subjecting the alloy to a heat treatment while restrained in the deformed configuration, the alloy could be deformed beyond a particular desired configuration and then heat treated such that there is a thermally induced change in configuration of the kind discussed below, the change taking the configuration towards the particular desired configuration. Introduction of the superelastic properties might also involve annealing at high temperature (for example towards the recrystallisation temperature of the alloy), followed by rapid cooling and then a heat treatment at a lower temperature.

The properties of shape memory alloys can also involve thermally induced changes in configuration in which an article is first deformed from a heat-stable configuration to a heat-unstable configuration while the alloy is in its martensite phase. Subsequent exposure to increased temperature results in a change in configuration from the heat-unstable configuration towards the original heat-stable configuration as the alloy reverts from its martensite phase to its austenite phase. It is known from US-5197978 to make use of the thermally induced change in configuration of an article made from a shape memory alloy in a stent.

Stents can also be made from materials that do not exhibit the shape memory properties of shape memory alloys. Examples include certain stainless steels.

Stents frequently have an apertured or open configuration which facilitates the controlled transverse compression and then outward recovery in use into contact with the wall of a lumen. The apertured configuration can comprise slits, or bigger openings. A stent with an apertured configuration can be formed directly by cutting a tube, or by cutting tube and then subjecting it to processes, to increase its diameter and to reset the increased diameter configuration as the heat stable configuration. It might also be formed from wire using an appropriate bonding technique at points where wires cross.

The configuration of the apertures in the stent will be selected to provide appropriate deformation characteristics, on both transverse compression prior to use and subsequently when the stent is disposed in a lumen. The configuration should also provide appropriate flexibility for the stent, prior to and during use. It is particularly desired that (a) the flexibility of the stent when bent relative to its longitudinal axis should be high, (b) the stent should be able to recover elastically from transverse compression, for example changing its configuration from elliptical to say circular, and (c) the radial stiffness of the stent should be high.

EP-A-669114 discloses a stent which comprises a plurality of aligned hoop portions, with adjacent hoop portions being connected to one another by means of at least one strut. The spacing between the hoop portions may vary along the length of the stent.

The present invention provides a stent according to claim 1.

Preferably, the spacing between adjacent hoop portions is greater at a point between the ends of the stent that at at least one of the ends. For example, the spacing between adjacent hoop portions can be greatest at a point along the length of the stent approximately midway between its ends. This general arrangement of the hoop portions allows the stent to be more flexible in a central portion along its length than at its ends. It can also be used in a lumen at a junction with another lumen, minimising resistance to flow of fluid between the lumen in which the stent is positioned and the other lumen, through the openings between the hoop portions.

The spacing between adjacent hoop portions can be greater at at least one of the ends of the stent than the spacing at a point between the said ends, for example at a point between the ends. Preferably, the spacing between adjacent hoop portions is greater at both ends of the stent that the spacing at a point between the ends. This general arrangement has the advantage that the flexibility of the stent can be arranged to be greater at its ends than at a point between its ends. This arrangement can therefore provide a degree of strain relief for the lumen in which the stent is positioned in use, which can be important when the lumen is delicate and susceptible to damage by a stent which exerts a high force on it.

Preferably, the ratio of the distance between the two most widely spaced hoop portions to the distance between the two closest spaced hoop portions is at least about 1.25, more preferably at least about 1.5, for example at least about 2.0.

Preferably, the distance between the two most widely spaced hoop portions to the distance between the two closest spaced hoop portions is not more than about 5, more preferably not more than about 4, for example not more than about 3.

The stent can include several regions with different spacings between adjacent hoop portions. For example, there might be regions with wide hoop spacings at one or both ends of the stent, or regions with wide hoop spacings at a point between the ends (for example for use at a junction between lumina), or both.

The stent of the invention has been found to provide an advantageous combination of properties. The hoop portions can provide firm support for a lumen against transverse compressive forces once the stent has been put in place, and can also exert firm and controlled outward pressure against the wall of the lumen when the stent expands. However, the nature of the strut connections between the hoop portions makes the stent flexible so that it can easily be bent. This can be useful when the stent is used in a lumen which flexes. Also, importantly, the ability of the stent to flex means that stresses applied against the walls of the lumen at the ends of the stent can be kept low (especially when the stent is inserted into a lumen which follows a tortuous path), resulting in reduced damage to the lumen at these locations (for example by restenosis of a blood vessel). However, a significant benefit flowing from the use of a flexible stent is that delivery of the stent to a particular location, often achieved by means of a catheter which passes through one or more lumens, is facilitated since the stent can flex as the catheter encounters obstructions or bends along its path. The flexibility of the stent can therefore simplify steering the catheter through the body to the desired location.

Preferably, the struts of the first hoop portion pair are distributed substantially uniformly around the stent, the struts of the second hoop portion pair preferably being arranged circumferentially around the stent between the struts of the first hoop portion pair. For example, when the stent is circular in cross-section and there are two struts extending between the hoop portions of the first hoop portion pair, they will be located at 0° and 180° around the hoop portions. When there are three struts extending between the hoop portions of the first hoop portion pair, they will be located at 0°, 120° and 240° around the hoop portions. Uniform arrangements of the struts when there are more then three between a pair of hoop portions will be apparent.

The number of struts between the hoop portions of the first hoop portion pair can be the same as between the hoop portions of the second hoop portion pair. Preferably, the struts between the hoop portions of the first pair are located symmetrically with respect to the struts between the hoop portions of the second pair. For example, if there are two struts between the hoop portions of the first and second pairs, those of one .pair will be located at 0° and 180° around the perimeter of the stent and those of the second pair will be located at 90° and 270°.

Preferably, there are at least two struts extending between the hoop portions of the first hoop portion pair, for example two, three or four struts. Most preferably, there are two struts between at least most of the pairs of adjacent hoop portions.

Preferably, the strut pattern differs between pairs of hoop portions of the stent. The pattern will therefore differ across a hoop portion between the struts to its neighbouring hoop portion on one side and those to the hoop portion on its other side. The flexibility of the stent can be optimised in this way. However, it can be appropriate for some applications for the strut pattern not to differ between opposite sides of all of the hoop portions, for example when it is desired to make the stent more flexible in one region than in another. Such increased flexibility can be achieved for example by incorporating extra struts between selected pairs of adjacent hoop portions. Struts can be provided in closely spaced multiples, for example two or more closely spaced struts, to provide increased stiffness in at least a region of the stent.

The struts used in the stent can be selected to provide desired effects on the flexibility of the stent. For example, the cross-section of the struts might differ from one strut to another; two or more strut portions might be provided closely spaced to one another to provide a multiple strut with relatively greater rigidity, the rigidity being affected by the cross-section of the strut portions and their spacing; one or more struts might be bent, to increase rigidity

An additional advantage of the stent design provided by the present invention is that it can be collapsed conveniently to reduce its transverse dimension, to facilitate delivery to the desired location in the lumen. This can conveniently be achieved by deforming the hoop portions from a planar configuration (with the planes extending transverse to the axis of the stent). This might be achieved for example by applying an axial force to the hoop portions at points between the struts. It will be understood that the references to hoop portions in the stent of the invention includes the hoop portions when in the planar configuration and when in a deformed configuration which is adopted for example when the stent is deformed for delivery. When the hoop portions are deformed from their planar configuration, they will tend towards that planar configuration when they recover, in use outwardly to support a lumen.

Preferably, the hoop portions of the stent, and preferably also the struts, are formed from a shape memory alloy, especially one which has been treated so that it exhibits enhanced elastic properties. In this way, the configuration of the stent can be changed between one in which it is compressed radially for insertion into a lumen and one in which it has a greater transverse dimension in which it can support a lumen and exert an outward force on it. Such alloys include binary alloys, such as those in which the nickel content is at least about 50 at.%, preferably at least about 50.5 at.%. The nickel content will usefully be less than about 52 at.%, preferably less than about 51 at.%. The stent can be formed from other Ni-Ti based alloys, including alloys with ternary and quaternary additions. Examples of elements that can be incorporated in the alloy include Fe, Co, Cr, Al, Cu and V. Added elements can be present in amounts upto about 10 at.%, preferably upto about 5 at.%.

The formation of the hoop portions from a shape memory alloy particularly assists in this transverse compression, the hoop portions being folded as the stent is compressed so that they become non-planar (which includes a change from a configuration in which the hoop portions are non-planar before compression to one in which their deviation from planarity is increased). A preferred technique for compressing the stent in this way involves applying axial force to the stent in opposed directions at spaced apart points around the hoop portions. The number and spacing of the points will depend on the number and arrangement of the struts which link the hoop portions together. For example, if there are two struts linking a pair of hoop portions, the hoop portions can be deformed by applying a force to the hoop portions in one axial direction at 0 and 180°, and a force in the opposite axial direction at 90 and 270°.

Preferably, the stent of the invention is made by a process which involves removing material from a sheath-like object, leaving a pattern of material with appropriate hoop portions and struts. The nature of the removal process will depend on the material of the sheath-like object. For example, the removal process may involve one or more of cutting, melting and vaporising the material. When the stent is formed from a metal material, the removal process can involve use of a laser cutting tool. Other techniques which might be used for forming the pattern in the material include stamping, cutting, and etching (especially photoetching).

The sheath-like object from which the stent is formed can be a tubular object, especially a cylindrical tube with a circular cross-section. However, the sheath can be filled with a core material. The core can support the sheath during the removal process. This can prevent or at least restrict deformation of the sheath during the removal process, and damage to the opposite side of the sheath from the point at which it is being cut by an external cutting tool. The core can be provided as a rod which can be slid into the sheath. The core and the sheath might be formed as a single article, for example by a cold drawing technique.

While the removal process referred to above is preferred for forming the stent of the invention, it might be formed in other ways, for example from wire by welding. The stent could also be made from sheet material which can be formed into a tube, for example by folding and welding.

Preferably, the stent is formed in such a way that the hoop portions and the struts are rigidly connected to one another at the points at which they intersect so that relative movement of a hoop portion and a strut requires deformation of the material by which the hoop portion and the strut are connected to one another. They might be rigidly connected to one another by means of a material other than the materials of the strut and the hoop portion, for example by means of an adhesive or a solder. They might be connected to one another without any other material, for example by means of welding or by forming the hoop portion and the strut from a single component for example by machining.

Preferably, the wall thickness of the material of the stent less than about 1.5 mm, more preferably less than about 0.8 mm. Preferably, the wall thickness is at least about 0.1 mm, more preferably at least about 0.2 mm.

Preferably, the maximum transverse dimension (which will be its diameter when the stent has a circular cross-section) of the stent (which will be its diameter when the stent has a circular cross-section) is not more than about 40 mm, more preferably not more than about 20 mm, especially not more than about 10 mm. Preferably, its minimum transverse dimension is at least about 0.5 mm, more preferably at least about 1 mm.

The invention is defined in the claims. The following might contain references to examples which are included in the description for background purposes only.

The present invention will now be described, by way of example only, with reference to the accompanying drawings, in which:
Figure 1 (a) to (d) are isometric views of stents in their configuration towards which they tend to expand elastically in use, to exert outward force on a lumen in which they are inserted, wherein the struts between the hoop portions are offset circumferentially.
Figure 2 is an isometric view of the stent shown in Figure 1 (a), in the transversely compressed configuration to which it is deformed elastically for insertion into the lumen, for example using a delivery tool such as a catheter, with an illustration of how it can be deformed to that configuration.
Figure 3 is an isometric view of the stent shown in Figure 1 (a), with an illustration of an alternative technique by which it can be deformed to the compressed configuration for insertion into the lumen.
Figure 4 is an isometric view of a stent in which the spacing between hoop portions varies between the ends and a point midway along its length.
Figure 5 is an isometric view of another stent in which the spacing between hoop portions varies between the ends and a point midway along its length.

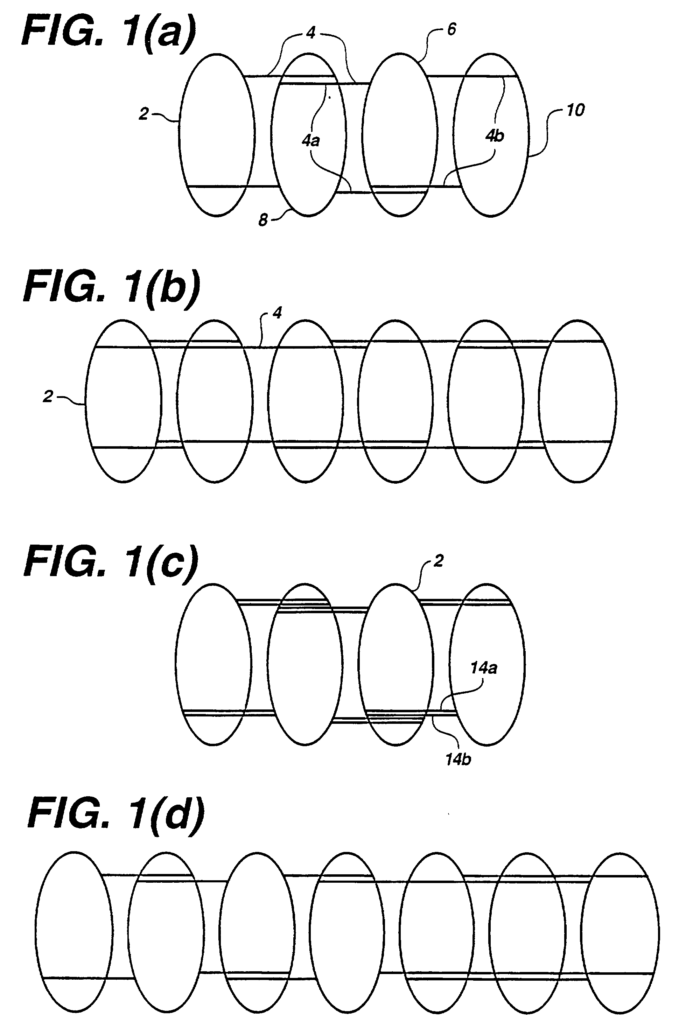

Referring to the drawings, Figure 1 shows four stents in their heat stable configuration. The stents of figure 1 and the following description illustrate the advantages provided by the feature of offset struts utilised by the present invention. Each comprises a plurality of hoop portions 2. The hoop portions are interconnected by means of struts 4. The struts 4a between one 6 of the hoop portions and its neighbouring hoop portion 8 on one side are offset circumferentially relative to the struts 4b between that hoop portion and its neighbouring hoop portion 10 on the other side. The offset struts facilitate bending of the stents relative to their longitudinal axes.

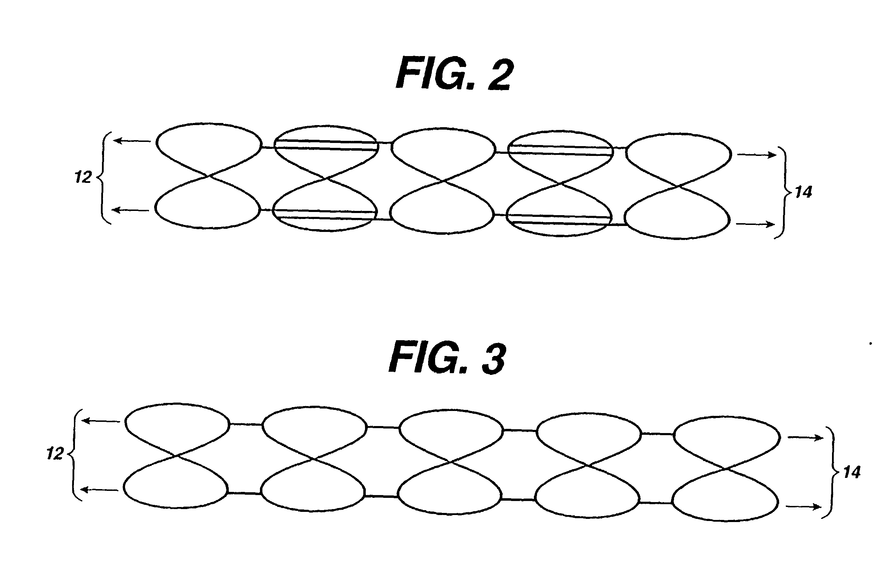

Figure 2 shows the stent shown in Figure 1 (a) in the transversely compressed configuration to which it is deformed elastically for insertion into the lumen, for example using a delivery tool such as a catheter. A preferred technique for making the stent shown in Figure 2 involves producing a tubular precursor from a shape memory alloy in the expanded configuration from which the compressed configuration shown in Figure 2 can be reached by transverse compression. The tubular precursor can be cut while in the expanded configuration to produce the structure of struts and hoop portions, for example using a laser cutting tool. Alternatively, the stent could be formed initially in the compressed configuration and then reset to the open configuration by heat treatment under constraint.

The stents shown in Figures 1 (a) and 2 are made from a binary Ni-Ti alloy which can have shape memory properties. The nature of the treatment of the stent to give it appropriate elastic properties will be apparent to a person skilled in the art.

The expanded stent preferably exists in its austenite phase. From this condition, the stent can be treated in different ways to make it suitable for use as a stent, where it will be inserted into a lumen (such as a blood vessel of a human or animal) in a transversely compressed configuration and then expanded. The stent can be subjected to a compression step in which it is compressed transversely towards the configuration of the stent shown in Figure 2 while at a temperature that is greater than Mₛ and less than M_{d} (the maximum temperature at which a phase change in the alloy from austenite to martensite can be induced by the application of stress) or, more preferably for some applications, at a temperature which is less than Mₛ. The stent can then be positioned within a constraint such as an artificial lumen (especially a delivery catheter) which retains the stent in its compressed configuration until it is to be allowed to expand in situ, towards the configuration after the expansion step.

The deformation of the stent from the configuration shown in Figure 1 (a) (or a configuration in which the hoop portions are substantially planar, or less non-planar than in the configuration shown in Figure 2) can be achieved by bending the hoop portions by applying axial forces to each hoop portion in opposite directions at spaced apart portions around the perimeter of the hoop portions, as shown by the arrows 12, 14, so as to collapse the hoop portions by folding them. These arrows are shown for the stent that is illustrated in Figure 1 (a). A similar technique but with forces applied at different points on the hoop portions can be used for other configurations of stents, such as the other configurations shown in Figure 1.

Another technique for bending the hoop portions to reduce the transverse dimension of the stent is shown in Figure 3 in which the stent is made to collapse by stretching it, by applying the axial force to the hoop portions at opposite ends of the stent, at two opposite points on each of the end hoop portions, the points being offset by 90° with respect to one another between the two opposed ends.

Figures 1 (b) to (d) show configurations of stents in which the arrangement of the struts 4 differs from that in the stent shown in Figure (a). In Figure 1 (b), struts extend across more than just two hoops, the struts being offset axially with respect to one another rather than having a pair of diametrically opposed struts extending between the same hoop portions.

In Figure 1 (c), struts are provided in circumferentially spaced pairs (for example 14a, 14b), with two pairs of struts in the illustrated arrangement. The use of struts in pairs (or larger groups) can provide increased resistance to bending of the stent. Changing the spacing of the paired struts becomes a simple and effective way of varying the stiffness of the stent along its length.

In Figure 1 (d), the pattern of struts is varied along the stent, with pairs of short struts towards one end and longer struts towards the other end, to provide differences in bending characteristics along the length of the strut.

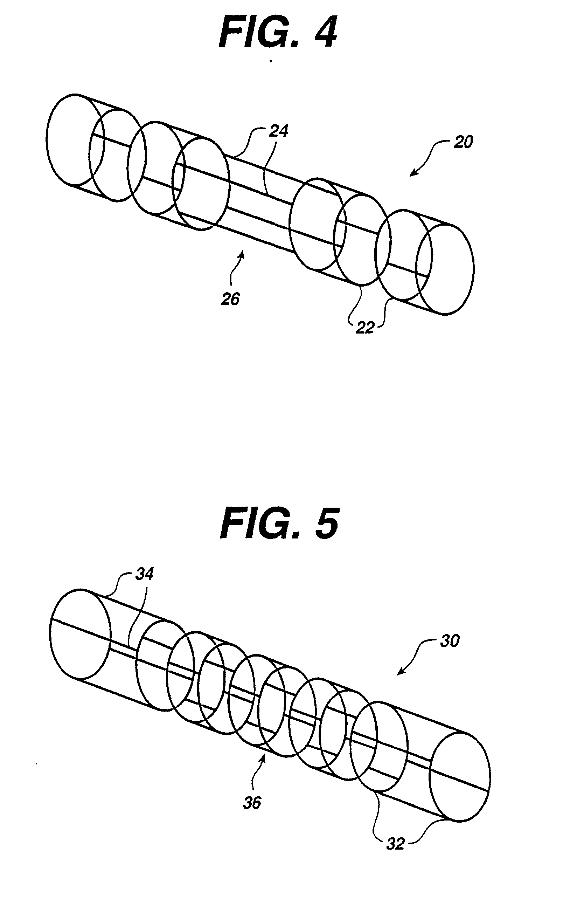

Figure 4 shows a stent 20 which comprises hoop portions 22 interconnected by struts 24. The spacing between the hoop portions in a region 26 between the ends of the stent is greater than the spacing between the hoop portions at the ends of the stent. The ratio of the distance between the two most widely spaced hoop portions at a midpoint to the distance between two closely spaced hoop portions at one of the ends of the stent is about 3. The stent 20 can be positioned in a lumen at a junction with another lumen, with the region 26 at the location of the junction. The wide spacing of the hoop portions in the region 26 of the stent at the junction facilitates flow of fluids between the lumina at the junction.

Figure 5 shows a stent 30 which comprises hoop portions 32 interconnected by struts 34. The spacing between the hoop portions in a region 36 between the ends of the stent is less than the spacing between the hoop portions at the ends of the stent. The ratio of the distance between the two closely spaced hoop portions at a midpoint to the distance between two widely spaced hoop portions at one of the ends of the stent is about 3. The wide spacing between the hoop portions at the ends of the stent increases the flexibility of the stent at its ends and minimises the strain exerted on a lumen by the stent when flexed.

## Claims

1. A stent comprising a plurality of aligned hoop portions (2, 8, 10), having a susbtantially planar configuration, wherein adjacent hoop portions are connected to one another by means of at least one strut (4, 4a), the strut (4a) between one hoop portion (6) and its neighbouring hoop portion (8) on one side, which together form a first hoop portion pair, being offset circumferentially relative to the strut (4b) between the said one hoop portion (6) and its neighbouring hoop portion (10) on the other side, which together form a second hoop portion pair, in order to increase the flexibility of the stent, wherein the spacing between the hoop portions is varying along the length of the stent.

2. A stent as claimed in claim 1, in which the spacing between adjacent hoop portions (2) is greater at a point between the ends of the stent that at at least one of the ends.

3. A stent as claimed in claim 2, in which the spacing between adjacent hoop portions (2) is greatest at a point along the length of the stent approximately midway between its ends.

4. A stent as claimed in claim 1, in which the spacing between adjacent hoop portions (2) is greater at at least one of the ends of the stent than the spacing approximately midway between the said ends.

5. A stent as claimed in claim 2, in which the spacing between adjacent hoop portions (2) is greater at both ends of the stent that the spacing approximately midway between its ends.

6. A stent as claimed in claim 1, in which the ratio of the distance between the two most widely spaced hoop portions (2) to the distance between the two closest spaced hoop portions is at least about 1.5.

7. A stent as claimed in claim 1, in which the ratio of the distance between the two most widely spaced hoop portions (2) to the distance between the two closest spaced hoop portions is not more than about 4.

8. A stent as claimed in claim 1, in which there are at least two struts (4) connecting adjacent hoop portions to one another.

9. A stent as claimed in claim 8, in which the struts (4a) of the first hoop portion pair are distributed substantially uniformly around the stent, the struts (4b) of the second hoop portion pair being arranged circumferentially around the stent between the struts of the first hoop portion pair.

10. A stent as claimed in claim 1, in which the number of struts (4a) extending between the hoop portions of the first hoop portion pair is the same as the number of struts (4b) extending between the hoop portions of second hoop portion pair.

11. A stent as claimed in claim 1, in which at least the hoop portions (2) are formed from a shape memory alloy.

12. A stent as claimed in claim 12, in which the hoop portions (2) and the struts (4) are formed from a shape memory alloy.

13. A stent as claimed in claim 12, in which the shape memory alloy has been treated so that it exhibits superelastic properties.

14. A stent as claimed in claim 1, which is in a radially compressed configuration, in which the hoop portions (2) are folded so that they are non-planar.

15. A stent as claimed in claim 14, which is held by a restraint in the said radially compressed configuration against recovery forces with which the stent would, but for the restraint, revert towards a configuration in which the hoop portions are substantially planar.

16. A stent as claimed in claim 1, in which the configuration of the struts (4) extending between a hoop portion (2) and its neighbouring portion on one side is different from the configuration of struts between that hoop portion and its neighbouring portion on the other side.

17. A stent as claimed in claim 1, in which at least two closely spaced struts extend between a pair of hoop portions to provide a multiple strut between those hoop portions.

18. A method of making a stent as claimed in any one of claims 1 to 17, which includes the step of removing material from a sheath-like object leaving a pattern of material with appropriate hoop portions and struts.

## Patentansprüche

1. Stent mit einer Vielzahl ausgerichteter Ringteile (2, 8, 10) mit im wesentlichen ebener Gestalt, wobei benachbarte Ringteile untereinander durch mindestens eine Strebe (4, 4a) verbunden sind und die Strebe (4a) zwischen einem Ringteil (6) und seinem auf einer Seite benachbarten Ringteil (8), welche zusammen ein erstes Ringteilpaar bilden, umfangsmäßig gegenüber der Strebe (4b) zwischen dem einem Ringteil (6) und seinem auf der anderen Seite benachbarten Ringteil (10), welche zusammen ein zweites Ringteilpaar bilden, versetzt ist, um die Flexibilität des Stents zu erhöhen und wobei der Abstand zwischen den Ringteilen entlang des Stents variiert.

2. Stent nach Anspruch 1, bei welchem der Abstand zwischen benachbarten Ringteilen (2) an einem Punkt zwischen den Enden des Stents größer ist als an mindestens einem der Enden.

3. Stent nach Anspruch 2, bei welchem der Abstand zwischen benachbarten Ringteilen (2) an einem Punkt entlang des Stents, etwa in der Mitte zwischen beiden seiner Enden, am größten ist.

4. Stent nach Anspruch 1, bei welchem der Abstand zwischen benachbarten Ringteilen (2) an mindestens einem der Enden des Stents größer ist als der Abstand etwa in der Mitte zwischen den Enden.

5. Stent nach Anspruch 2, bei welchem der Abstand zwischen.benachbarten Ringteilen (2) an seinen beiden Enden größer ist als der Abstand etwa in der Mitte zwischen beiden seiner Enden.

6. Stent nach Anspruch 1, bei welchem das Verhältnis des Abstandes zwischen den beiden Ringteilen (2) mit dem größten Abstand zum Abstand zwischen den Ringteilen mit dem geringsten Abstand mindestens 1,5 beträgt.

7. Stent nach Anspruch 1, bei welchem das Verhältnis des Abstandes zwischen den beiden Ringteilen (2) mit dem größten Abstand zum Abstand zwischen den Ringteilen mit dem geringsten Abstand nicht mehr als 4 beträgt.

8. Stent nach Anspruch 1, bei welchem mindestens zwei Streben (4) benachbarte Ringteile miteinander verbinden.

9. Stent nach Anspruch 8, bei welchem die Streben (4a) des ersten Ringteilpaares im wesentlichen gleichmäßig rund um den Stent verteilt sind und die Streben (4b) des zweiten Ringteilpaares rund um den Stent zwischen den Streben des ersten Ringteilpaares angeordnet sind.

10. Stent nach Anspruch 1, bei welchem die Anzahl der Streben (4a), die sich zwischen den Ringteilen des ersten Ringteilpaares erstreckt, gleich der Anzahl der Streben (4b) ist, die sich zwischen den Ringteilen des zweiten Ringteilpaares erstreckt.

11. Stent nach Anspruch 1, bei welchem zumindest die Ringteile (2) aus einer Formgedächtnislegierung hergestellt sind.

12. Stent nach Anspruch 11, bei welchem zumindest die Ringteile (2) und die Streben (4) aus einer Formgedächtnislegierung hergestellt sind.

13. Stent nach Anspruch 12, bei welchem die Formgedächtnislegierung derart behandelt wurde, daß sie superelastische Eigenschaften aufweist.

14. Stent nach Anspruch 1, welcher eine radial zusammengedrückte Anordnung aufweist, in welcher die Ringteile (2) derart gefaltet sind, daß sie nicht eben sind.

15. Stent nach Anspruch 14, welcher zwangsweise, entgegen Rückfederungskräften in der radial zusammengedrückten Anordnung, gehalten wird, aus welcher er bei Wegfall des Zwanges in eine Anordnung zurückkehrt, in welcher die Ringteile im wesentlichen eben sind.

16. Stent nach Anspruch 1, bei welchem sich die Anordnung der Streben (4), die sich zwischen einem Ringteil (2) und seinem auf einer Seite benachbarten Ringteil erstrecken, von der Anordnung der Streben zwischen diesem Ringteil und seinem auf der anderen Seite benachbarten Ringteil unterscheidet.

17. Stent nach Anspruch 1, bei welchem sich mindestens zwei Streben mit geringem Abstand zwischen einem Ringteilpaar erstrecken, um zwischen diesen Ringteilen eine Mehrfachstrebe zu bilden.

18. Verfahren zur Herstellung eines Stents nach einem der Ansprüche 1 bis 17 mit dem Schritt der Entfernung von Material aus einem hülsenförmigen Gegenstand derart, daß eine Materialanordnung mit geeigneten Ringteilen und Streben zurückbleibt.

## Revendications

1. Extenseur comprenant une pluralité de parties de boucle alignées (2, 8, 10) possédant une configuration sensiblement plane, les parties de boucle adjacentes étant connectées entre elles par au moins une entretoise (4, 4a), l'entretoise (4a) étant disposée entre une partie de boucle (6) et la partie de boucle (8) voisine d'un côté, les deux parties de boucle formant une première paire de partie de boucle, en étant décalées circonférentiellement par rapport à l'entretoise (4b) entre ladite partie de boucle (6) et la partie de boucle (10) voisine de l'autre côté, ces parties de boucles formant conjointement une seconde paire de partie de boucle de manière à augmenter la flexibilité de l'extenseur, l'espacement entre les parties de boucle étant variable sur la longueur de l'extenseur.

2. Extenseur selon la revendication 1, dans lequel l'espacement entre des parties de boucles adjacentes (2) est élevé en un point situé entre les extrémités de l'extenseur qu'à au moins l'une des extrémités.

3. Extenseur selon la revendication 2, dans lequel l'espacement entre des parties de boucles adjacentes (2) est maximal en un point qui est situé sur la longueur de l'extenseur approximativement à mi-chemin entre ces extrémités.

4. Extenseur selon la revendication 1, dans lequel l'espacement entre des parties de boucles adjacentes (2) est plus important en au moins une des extrémités de l'extenseur que l'espacement approximativement intermédiaire entre lesdites extrémités.

5. Extenseur selon la revendication 2, dans lequel l'espacement entre des parties de boucles adjacentes (2) est plus important au niveau des deux extrémités de l'extenseur que l'espacement approximativement à mi-chemin entre ces extrémités.

6. Extenseur selon la revendication 1, dans lequel le rapport de la distance entre les deux parties de boucles (2) qui sont les plus espacées l'une de l'autre à la distance entre les deux parties de boucles qui sont les moins espacées est égal au moins à environ 1,5.

7. Extenseur selon la revendication 1, dans lequel le rapport de la distance entre les deux parties de boucles les plus espacées (2) à la distance entre les deux parties de boucles les plus proches n'est pas supérieur à environ 4.

8. Extenseur selon la revendication 1, dans lequel il est prévu au moins deux entretoises (4) reliant des parties de boucles adjacentes entre elles.

9. Extenseur selon la revendication 8, dans lequel les entretoises (4a) de la première paire de partie de boucle sont distribuées sensiblement d'une manière uniforme autour de l'extenseur, les entretoises (4b) de la seconde paire de parties de boucles étant disposées circonférentiellement autour de l'extenseur entre les entretoises de la première paire de partie de boucle.

10. Extenseur selon la revendication 1, dans lequel le nombre d'entretoises (4a) s'étendant entre les parties de boucles de la première paire de partie de boucle est égal au nombre d'entretoises (4b) s'étendant entre les parties de boucles de la seconde paire de partie de boucle.

11. Extenseur selon la revendication 1, dans lequel au moins les parties de boucles (2) sont réalisées en un alliage à mémoire de forme.

12. Extenseur selon la revendication 12, dans lequel les parties de boucles (2) et les entretoises (4) sont réalisées en un alliage à mémoire de forme.

13. Extenseur selon la revendication 12, dans lequel l'alliage à mémoire de forme a été traité de manière à présenter des propriétés superélastiques.

14. Extenseur selon la revendication 1, qui est dans une configuration comprimée radialement, dans laquelle les parties de boucles (2) sont repliées de telle sorte qu'elles ne sont pas planes.

15. Extenseur selon la revendication 14, qui est retenu par une contrainte dans ladite configuration comprimée radialement à l'encontre de forces de rappel avec lesquelles l'extenseur serait ramené, s'il n'y avait la contrainte, à une configuration dans laquelle les parties de boucles sont sensiblement planes.

16. Extenseur selon la revendication 1, dans lequel la configuration des entretoises (4), qui s'étendent entre une partie de boucle et sa partie voisine d'un côté diffère de la configuration d'entretoises entre cette partie boucle et sa partie voisine, de l'autre côté.

17. Extenseur selon la revendication 1, dans lequel au moins deux entretoises faiblement espacées s'étendent entre une paire de parties de boucle pour former une entretoise multiple entre ces parties de boucles.

18. Procédé pour fabriquer un extenseur selon l'une des quelconques revendications 1 à 17, qui inclut l'étape consistant à enlever du matériel d'un objet en forme de gaine en laissant subsister une configuration de matériel comportant des parties de boucles et des entretoises appropriées.
